# EUROPEAN PATENT SPECIFICATION

(11) **EP 1 996 131 B1**
(45) Date of publication and mention of the grant of the patent: **12.04.2017**
(21) Application number: 07757555.3
(22) Date of filing: 27.02.2007
(51) Int. Cl.: A61F 2/95, A61F 2/966

(54) **IMPLANTABLE MEDICAL ENDOPROSTHESIS DELIVERY SYSTEMS**
IMPLANTIERBARE MEDIZINISCHE SYSTEME ZUR VERABREICHUNG VON ENDOPROTHESEN
SYSTÈMES DE LIVRAISON D'ENDOPROTHÈSE MÉDICALE IMPLANTABLE

(30) Priority: 06.03.2006 US 368572
(43) Date of publication of application: 03.12.2008
(73) Proprietor: Boston Scientific Limited, Hamilton HM11 (BM)
(72) Inventor: GUNDERSON, Richard C., Maple Grove, MN 55311 (US)
(74) Representative: Vossius & Partner Patentanwälte Rechtsanwälte mbB
(86) International application number: PCT/US2007/062884
(87) International publication number: WO 2007/103666

(56) References cited:
- WO-A-2005/107644

## Description

### TECHNICAL FIELD

This invention relates to medical endoprosthesis delivery systems, as well as related components and methods.

### BACKGROUND

Systems are known for delivering medical devices, such as stents, into a body lumen. Often, such systems include a proximal portion that remains outside the body during use and a distal portion that is disposed within the body during use. The proximal portion typically includes a handle that is held by an operator of the system (e.g., a physician) during use, and the distal portion can include an outer member surrounding an inner member with a stent positioned therebetween. Generally, the operator of the system positions the distal portion within the lumen at a desired location (e.g., so that the stent is adjacent an occlusion). The operator can then retract the outer member to allow the stent to engage the occlusion/lumen wall. Thereafter, the operator removes the distal portion of the system from the lumen.
WO2005107644 A1 relates to a medical device for delivery of a self-expanding stent. The device comprises a catheter assembly having a catheter shaft and a self-expanding stent. A retractable sheath is disposed about the catheter shaft and the stent to retain the stent in a constricted diameter. A retractable membrane is also disposed about the catheter shaft and the stent prior to delivery. The retractable sheath and the retractable membrane are independently moveable relative to one another.

### SUMMARY

The present invention is defined by the features of the claims.

Implantable medical endoprosthesis delivery and deployment devices can include three members, a first or inner member, a second or middle member, and a third or outer member. An implantable medical endoprosthesis (e.g., a self-expanding stent, stent-graft, or graft) is typically located adjacent a distal end of the device, between the inner and middle members. In embodiments of the invention, a membrane can be connected to the middle and inner members and extends between the middle member and the endoprosthesis. The middle member is slidably disposed between the inner and outer members such that it can move longitudinally relative to the inner and outer members. Optionally, the inner and outer members are rigidly connected to one another such that there is relatively little (e.g., no) longitudinal movement between the inner and outer members.

In operation, the device is inserted into a body lumen (e.g., an artery of a human) and moved to a delivery site (e.g., adjacent an occlusion in an artery). The middle member is then retracted in a proximal direction, causing the membrane to roll or fold back upon itself and expose the endoprosthesis, which deploys. The outer member can remain stationary throughout the retraction and deployment of the middle member, allowing the delivery device to be held stationary during the deployment of the endoprosthesis, which can increase the accuracy of deployment of the endoprosthesis. With this design, the endoprosthesis is deployed without its outer surface being exposed to a sliding surface (e.g., the sliding inner surface of the retracting middle member). This can further increase deployment accuracy (e.g., by reducing the proximal forces on the endoprosthesis during delivery).

Embodiments can include one or more of the following features.

The systems can be designed so that there is relatively little relative longitudinal movement between the inner and outer members.

Prior to deployment, the distal end of the outer member can be sufficiently proximal to the pre-deployed endoprosthesis so that, during deployment, the endoprosthesis can expand to a diameter greater than that of the outer member as the middle member is retracted.

Embodiments may include one or more of the following advantages.

The membrane may enable deployment of stents, stent-grafts, grafts, and the like having a coating (e.g., a coating including a therapeutic agent) with little loss of the coating due to friction upon delivery.

The outer member and/or inner member (and optional bumper attached thereto) can be held substantially stationary, relative to the body in which the device is inserted, while the middle member is retracted, which can increase the accuracy of deployment of the endoprosthesis.

Other features and advantages of the invention will be apparent from the description, drawings and claims.

The following items are preferred embodiments of the invention.

### DESCRIPTION OF DRAWINGS

FIG 1 is a cross-sectional view of an embodiment of a system.
FIG 2 is a cross-sectional view of an embodiment of the system of FIG. 1.
FIG 3 is a cross-sectional view of an embodiment of the system of FIG. 1.
FIG 4 is a diagram of a physician using an embodiment of a system.
FIG 5 is a cross-sectional view of an embodiment of a system.
FIG 6 is a cross-sectional view of the embodiment of FIG. 5.
FIG. 7A is a cross-sectional view of an embodiment of a system.
FIG 7B is a cross-sectional view of an embodiment of a system.
FIG. 7C is a cross-sectional view of an embodiment of a system.
FIG 8 is a cross-sectional view of an embodiment of a system.
FIG 9 is a cross-sectional view of an embodiment of a system.
Like reference symbols in the various drawings indicate like elements.

### DETAILED DESCRIPTION

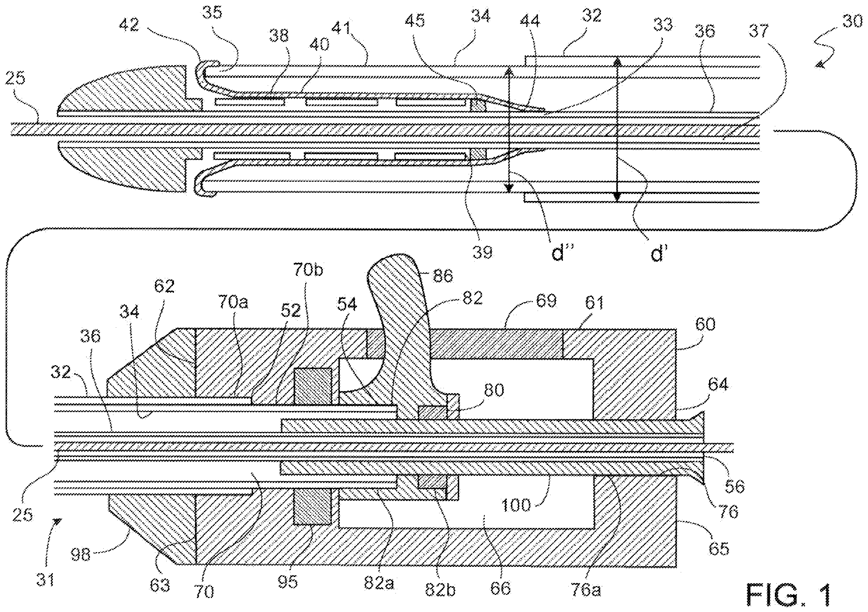

Referring now to FIG. 1, an embodiment of the delivery and deployment system is shown schematically in a delivery mode (i.e., before any deployment steps have occurred) and is generally referred to as 30, and includes an outer member 32, a middle member 34 and an inner member 36. The inner member 36 defines a lumen 37 that can accept a guide wire 25. A self-expanding stent 38 is shown in the delivery position in FIG 1, carried axially around the inner member 36 and held in its reduced delivery configuration by the middle member 34 in combination with a membrane 40. Also contained between the membrane 40 and the inner member 36 is a bumper 45, which is connected (e.g., by adhesive) to the inner member 36 at a position proximal to the stent 38. The bumper 45 can reduce (e.g., prevent) proximal movement of the stent 38 during deployment. The membrane 40 is connected at a first end 42 to a distal end 35 of the middle member 34, and at a second end 44 to a portion 33 of the inner member that is proximal to the first end 42 of the membrane.

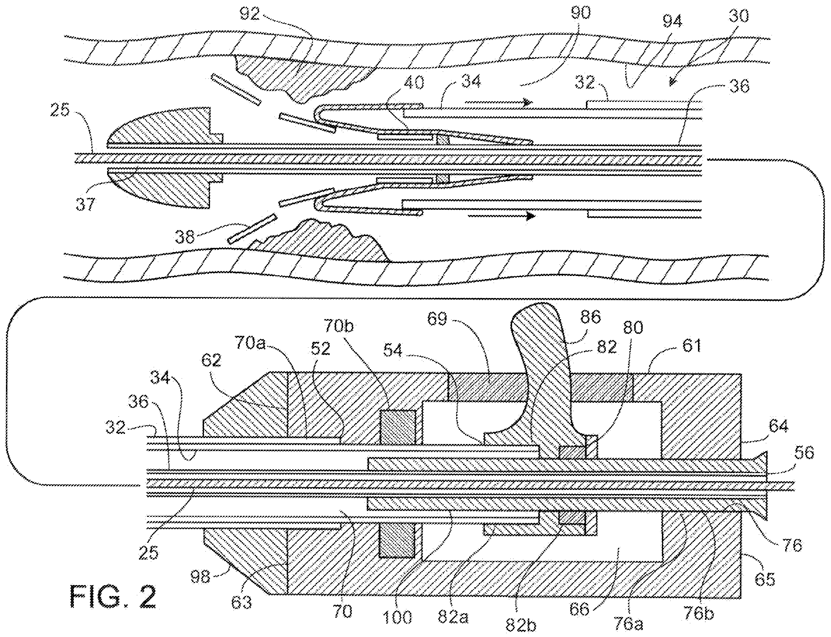

The outer member 32 extends distally to a point proximal of the distal end of the middle member 34 when the middle member 34 is in a deployment position (e.g., is retracted sufficiently for the stent 38 to deploy). This arrangement allows the stent 38 to expand upon retraction of the middle member 34 without the stent 38 contacting the outer member 32. The outer member 32 extends distally substantially all the way to a proximal end 39 of the endoprosthesis 38, so as to provide a barrier between the middle member 34 and the body lumen walls 94 (FIG. 2), which can reduce the friction of pull-back and lessen potential damage to the body lumen walls 94. Generally, the outer member 32 extends distally at least long enough to extend into an introducer sheath (not illustrated) during use. In some embodiments, the outer member 32 extends into but no further than the introducer sheath, potentially minimizing an outer diameter d" of the portion of the system 30 being threaded through the body lumen or lumens.

A handle 60 is attached to a proximal end 31 of the delivery device 30. The handle 60 has a body 61 having a distal end 62 and a proximal end 64. A recess 66 extends from a point proximal the distal end 62 of the handle 60 to a point distal the proximal end 64 of the handle 60. A distal orifice 70 extends from a distal face 63 through the handle to the recess 66. The distal orifice 70 has a first diameter 70***a*** at its distal end which is large enough to accommodate the outer member 32, and a second diameter 70***b*** at its proximal end large enough to slidably receive the middle member 34. A proximal orifice 76 extends from a proximal face 65 of the handle through the handle 60 to the recess 66. The proximal orifice 76 has a third diameter 76***a*** large enough to accommodate the inner member 36 and a hypotube 100, which extends into the recess 66 and optionally into the distal orifice 70. The hypotube 100 is fixed to the body 61 such that it cannot move longitudinally relative to the body 61.

A proximal end 52 of the outer member 32 extends into the distal orifice 70 in the handle body 61 and is attached to the body 61 within the first diameter 70***a*** of the distal orifice 70. A strain relief 98, typically formed of a non-rigid material (e.g., a relatively soft polymer or rubber) is connected to the distal end 62 of the handle body 61 and extends over the outer member 32 distally of the handle body 61. The strain relief can reduce the strain put onto the outer and/or middle members 36, 34, by the edges of the handle 60 (e.g., by reducing the degree to which the outer and/or middle members 34, 36 can bend relative to the handle 60).

A proximal end 54 of the middle member 34 extends through distal orifice 70 into the recess 66 of the handle body 61, where it is received by a pull-back actuator 80 that is slidably disposed within the recess 66. The middle member 34 can be connected to the pull-back actuator 80 by any conventional mechanism, including adhesive, chemical welding, mechanical connection, lap welding and/or butt welding. Optionally, the pull-back actuator 80 can be connected to the middle member 34 by being molded directly onto the middle member 34, e.g., by injection molding.

A proximal end 56 of the inner member 36 also extends through the distal orifice 70 into the recess 66, where it is received by and is fixed to the hypotube 100. The pull-back actuator 80 includes a bore 82 extending in a longitudinal direction and having a diameter sufficient to receive and slide over the hypotube 100. Optionally, the exterior of the hypotube 100 within the recess 66, the interior of the bore 82, or both, can have a lubricious coating applied thereto to improve the slidability of the pull-back actuator 80 over the hypotube 100.

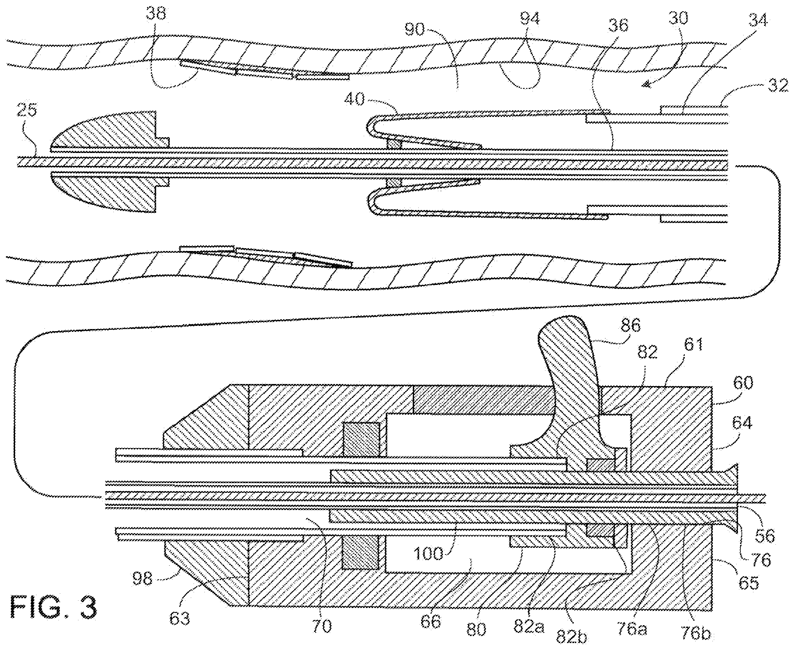

The result of this configuration is that the outer and inner members 32, 36 are attached to the body 61 such that their ability to move longitudinally relative to the body 61 is reduced, as is their ability to move longitudinally relative to one another. The middle member 34, attached to the pull-back actuator 80, can slide longitudinally as the pull-back actuator 80 is slid within the recess 66. In general, the recess 66 should extend sufficiently longitudinally to provide enough room for the middle member 34 to be retracted enough to completely release the stent 38 to self-expand, as shown in FIG. 3.

The pull-back actuator 80 includes a lever 86 that extends through a slot 69 in the body 61. The lever 86 permits the middle member 34 to be retracted using one hand (e.g., using a thumb) freeing the other hand for steadying the system 30 elsewhere, as explained in greater detail below.

In operation, as illustrated in FIGS. 1-3, the guide wire 25 is inserted into a body lumen 90 to a point at least slightly beyond a target deployment site. The device 30, in a delivery configuration (as in FIG. 1), is threaded over guide wire 25, such that the guide wire 25 extends through the lumen 37 of the inner member 36. The device 30 is then inserted into the body lumen to a point at which the stent 38 is located at the target deployment site, here having an occlusion 92, as illustrated in FIG. 2. The pull-back actuator 80 is then slid proximally within the recess 66 of the handle 60, partially retracting the middle member 34 and sliding the membrane 40 back upon itself to partially expose the stent 38, which begins to expand. When the pull-back actuator 80 is slid sufficiently far that the stent 38 is exposed, as in FIG. 3, the stent 38 expands to contact the walls of the body lumen 90.

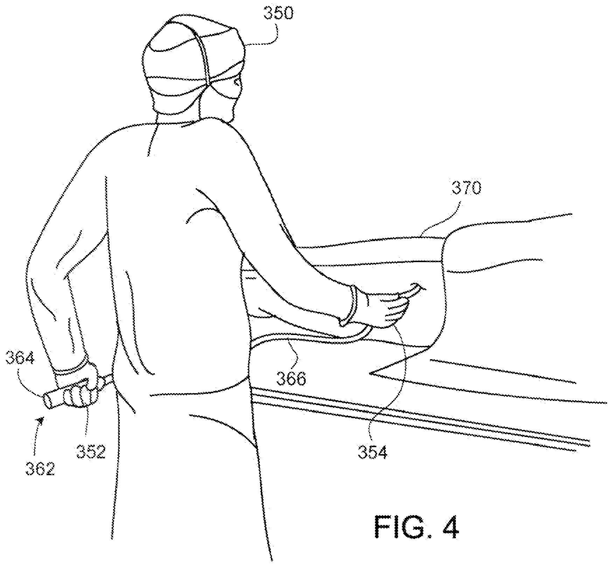

When the middle member 34 is being retracted, the operator can hold the delivery device steady by grasping the outer member 32, for example, at the point of entry into an introducer sheath or at or near the point of entry into the body. For example, as illustrated in FIG. 4, a physician 350 can grasp a handle 364 of a delivery system 362 with a first hand 352 and grasp the outer, stationary member 366 of the system 360 with a second hand 354 near the point of entry into the subject 370, thus reducing (e.g., preventing) the system 362 from movement (e.g., longitudinal movement) during the deployment of the endoprosthesis. The physician 350, by holding the outer member 366 motionless, can hold the inner member motionless; the bumper located proximal the endoprosthesis, in conjunction with the distal tip or optional distal bumpers, will then keep the endoprosthesis from longitudinal movement during deployment, which can result in more accurate placement of the endoprosthesis.

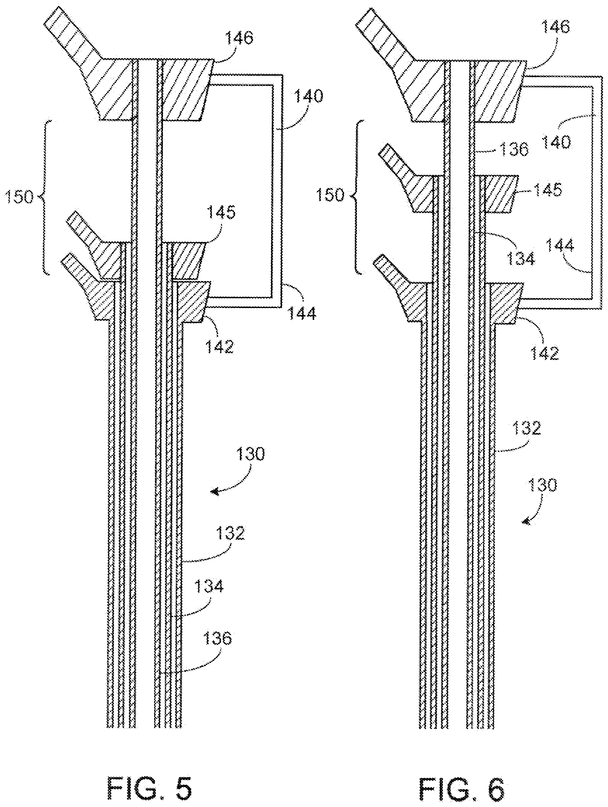

The proximal end of the device can vary, provided that the inner and outer members are unable to move longitudinally relative to one another. For example, in certain embodiments, illustrated in FIGS. 5 and 6, outer member 132 and inner member 136 of a device 130 are connected together by a manifold stabilizer 140, which includes an outer member handle 142 and an inner member handle 146 connected by a stabilizing member 144, at the proximal end of the device. Middle member 134 is connected at its proximal end to a separate pull-back handle 145, which can slide longitudinally in a gap 150 between the outer member handle 142 and the inner member handle 146. In general, the gap 150 should provide enough room for the middle member 134 to be fully retracted to permit the complete exposure and deployment of an endoprosthesis (not illustrated).

The handle can incorporate any known member retraction mechanism, for example, a pull-back handle or lever, a dial back system, a rack and pinion system, a ratchet system, a pulley system and/or a gear system.

Generally, the inner, middle and outer members can be formed of single wall tubing, braided tubing, braid-reinforced tubing, coil-reinforced tubing, multi-layer tubing, and/or precision cut tubing for flexibility. The inner member, the middle member, and/or outer member can be made of, for example, one or more polymers. Examples of polymers include polyether-block co-polyamide polymers (e.g., PEBAX^{®}), copolyester elastomers (e.g., Arnitel^{®} copolyester elastomers), thermoset polymers, polyolefins (e.g., Marlex^{®} polyethylene, Marlex^{®} polypropylene), high-density polyethylene (HDPE), low-density polyethylene (LDPE), polyamides (e.g., Vestamid^{®}), polyetheretherketones (PEEKs), and silicones. Other examples of polymers include thermoplastic polymers, such as polyamides (e.g., nylon), thermoplastic polyester elastomers (e.g., Hytrel^{®}), and thermoplastic polyurethane elastomers (e.g., Pellethane^{™}). The inner member, the middle member, and/or the outer member can include the same polymers and/or can include different polymers.

In certain embodiments, the inner and/or outer surface of the inner member, the middle member, and/or the outer member includes a lubricious coating or lining. For example, in certain embodiments, the inner member includes a guide wire lumen that is coated with a polymer (e.g., polytetrafluoroethylene (PTFE), polyimide, or high density polyethylene (HDPE)) that can decrease friction between the guide wire lumen and a guide wire that is disposed within guide wire lumen.

In some embodiments, one or more regions of the inner member and/or the outer member can be formed by an extrusion process. In some embodiments, different regions (e.g., different regions made up of different polymers) can be integrally formed. In certain embodiments, different regions can be separately formed and then connected together.

In certain embodiments, the inner member, the middle member, and/or the outer member can be formed of multiple layers. For example, one or more of the members can include three layers: an outer polymer layer, an inner polymer layer, and an intermediate structural layer disposed between the inner and outer layers. The inner polymer layer can be, for example, an HDPE or a PTFE, such as PTFE that has been etched on a surface that is to be bonded to the middle layer (e.g., to improve bonding to other layers). The intermediate structural layer can be, for example, a braid layer. In certain embodiments, the braid layer can be formed of a metal (e.g., tungsten) or metal alloy (e.g., stainless steel). In some embodiments, the braid layer can include one or more flat wires and/or one or more round wires. In certain embodiments, the braid layer can form a pattern between the inner layer and the outer layer. The outer polymer layer can be, for example, nylon, HDPE, PEBAX^{®}, Arnitel^{®}, or Hytrel^{®}.

In certain embodiments, the inner member, the middle member, and/or the outer member can have one or more translucent regions, or can be formed entirely of translucent material. In some embodiments, the inner member, the middle member, and/or the outer member can be formed of multiple polymer layers of differing durometers. In certain embodiments, the inner member, the middle member, and/or the outer member can include multiple coextruded layers. For example, an inner member with an inner layer including HDPE, an outer layer including PEBAX^{®}, and a tie layer between the inner and outer layers can be formed by coextrusion. Coextrusion processes are described in, for example, U.S. Patent Application Publication No. US 2002/0165523 A1, published on November 7, 2002, and U.S. Patent Application No. 10/351,695, filed on January 27, 2003, and entitled "Multilayer Balloon Member".

Certain of the above-described embodiments include a bumper, typically attached to or integral with the inner member at a position proximal the endoprosthesis. The bumper can reduce the possibility of the endoprosthesis moving proximally as outer member is retracted proximally. In some embodiments, the bumper is formed of a polymeric material, such as a polyether-block co-polyamide polymer (e.g., PEBAX^{®}) or a thermoplastic polyurethane elastomer (e.g., Pellethane^{™}). In certain embodiments, the bumper is made of a metal or an alloy, such as, for example, stainless steel, Nitinol and/or platinum.

The inner member can in certain embodiments have an inner diameter of no more than about 0.7 mm (e.g., no more than about 0.6 mm, no more than about 0.5 mm, no more than about 0.4 mm, or no more than about 0.3 mm) and/or no less than about 0.2 mm (e.g., no less than about 0.3 mm, no less than about 0.4 mm, no less than about 0.5 mm, or no less than about 0.6 mm mm). The inner diameter can be large enough to accommodate a wire (e.g., a guidewire) therethrough. For example, the inner diameter can be large enough to accommodate a guidewire having a diameter of no more than about 0.6 mm (e.g., no more than about 0.5 mm, no more than about 0.4 mm, or no more than about 0.3 mm). The inner member can in certain embodiments have an outer diameter of no more than about 1.2 mm (e.g., no more than about 1.1 mm, no more than about 1 mm, no more than about 0.9 mm, or no more than about 0.8 mm) and/or no less than about 0.7 mm (e.g., no less than about 0.8 mm, no less than about 0.9 mm, no less than about 1 mm, or no less than about 1.1 mm). The outer diameter can be sized to accept an endoprosthesis in a reduced configuration thereabout.

The middle member can in certain embodiments have an inner diameter of no more than about 1.5 mm (e.g., no more than about 1.4 mm, no more than about 1.3 mm, no more than about 1.2 mm, or no more than about 1.1 mm) and/or no less than about 1 mm (e.g., no less than about 1.1 mm, no less than about 1.2 mm, no less than about 1.3 mm, or no less than about 1.4 mm). The inner diameter can be large enough to accommodate the inner member therethrough, as well as the endoprosthesis and membrane at the distal end of the middle member. The middle member can in certain embodiments have an outer diameter of no more than about 1.8 mm (e.g., no more than about 1.7 mm, no more than about 1.6 mm, no more than about 1.5 mm, or no more than about 1.4 mm) and/or no less than about 1.3 mm (e.g., no less than about 1.4 mm, no less than about 1.5 mm, no less than about 1.6 mm, or no less than about 1.7 mm).

The outer member can in certain embodiments have an inner diameter just large enough to accept the middle member therein. In some embodiments, the inner diameter of the outer member is substantially the same as the outer diameter of the middle member. The inner diameter of the outer member can in other embodiments be large enough to accommodate the middle member therethrough and create a lumen between the middle member and the outer member to permit fluid flow (e.g., lubricious fluid flow) therethrough. The outer member can in certain embodiments have a diameter that is no more than about 0.6 mm larger (e.g., no more than about 0.5 mm larger, no more than about 0.4 mm larger, no more than about 0.3 mm larger, no more than about 0.2 mm larger, or no more than about 0.1 mm larger) and/or no less than about 0.05 mm larger (e.g., no less than about 0.1 mm larger, no less than about 0.2 mm larger, no less than about 0.03 mm larger, no less than about 0.4 mm larger, or no less than about 0.5 mm larger) than the outer diameter of the middle member. In some embodiments, the inner diameter of the outer member can be no more than about 1.9 mm (e.g., no more than about 1.8 mm, no more than about 1.7 mm, no more than about 1.6 mm, or no more than about 1.5 mm) and/or no less than about 1.4 mm (e.g., no less than about 1.5 mm, no less than about 1.6 mm, no less than about 1.7 mm, or no less than about 1.8 mm).

In certain embodiments, the outer member can have an outer diameter of no more than about 2.1 mm (e.g., no more than about 2.0 mm, no more than about 1.9 mm, no more than about 1.8 mm, or no more than about 1.7 mm) and/or no less than about 1.6 mm (e.g., no less than about 1.7 mm, no less than about 1.8 mm, no less than about 1.9 mm, or no less than about 2.0 mm mm).

The inner diameter, outer diameter, and/or wall thickness of one or more of the inner, middle and outer members need not be constant throughout the length of the member. For example, the middle member can have a larger inner diameter, and optionally a larger outer diameter, at the region which retains the endoprosthesis and membrane, and a reduced diameter proximal to that region.

The membrane in some embodiments is constructed at least in part of one or more of a variety of flexible materials, including, for example, thermoplastic elastomers including polyether block amides (e.g., PEBAX^{®}), polyethylenes (e.g., polyethylene terphthalate (PET)), nylon, ionomer (e.g., Surlyn^{®} ionomer), polyurethane, Arnitel^{®} copolyester elastomer, Hytrel^{®} thermoplastic elastomer, and/or blends thereof. Materials from which medical balloons are manufactured can be employed. In some embodiments the membrane may include a nanocomposite material, e.g., a nanoceramic material, which may add durability and/or lubricity. In some embodiments the membrane is at least partially made from one or more polymers with surface alterations (e.g., plasma treatment) for enhanced lubricity. In some embodiments the membrane is formed of more than one layer of material (e.g., two, three, four or five or more layers of material). In certain embodiments one or both sides of the membrane are coated and/or provided with surface enhancements (e.g., coated with silicones or other substances) to enhance lubricity.
The wall thickness of the membrane in some embodiments is no less than about 0.0254 mm (0.001 inch) (e.g., no less than about 0.0508 mm (0.002 inch), no less than about 0.0762 mm (0.003 inch), or no less than about 0.1016 mm (0.004 inch)) and/or no more than about 0.127 mm (0.005 inch) (e.g., no more than about 0.1016 mm (0.004 inch), no more than about 0.0762 mm (0.003 inch), no more than about 0.0508 mm (0.002 inch), or no more than about 0.0254 mm (0.001 inch)) thick. In selecting the wall thickness, account must be taken of the dimensions of the region of the device in which the membrane will reside; enough clearance must exist such that the membrane can be retracted off of the endoprosthesis.

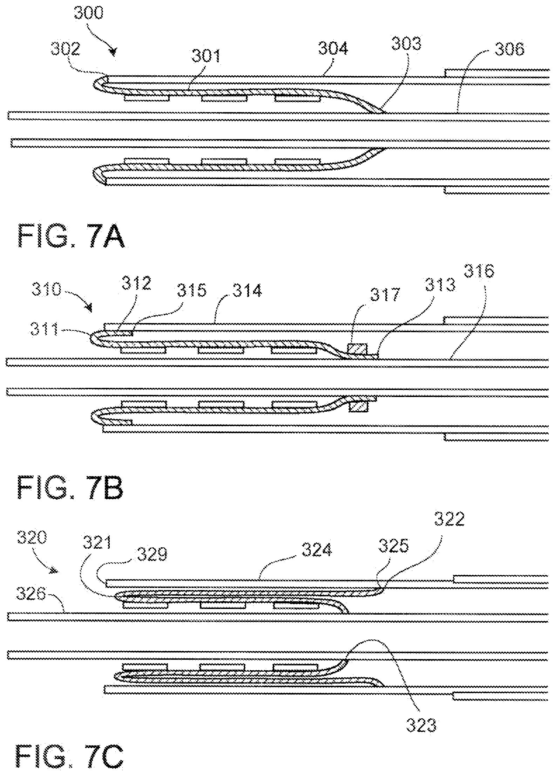

The membrane can be connected to the inner and middle members together by chemical or adhesive welding or bonding, fusion or heat welding, or ultrasonic welding; by mechanically engaging the membrane and the respective members along complementary surfaces; by an additional component such as a fastener or other device utilized to secure the components together; by butt-welding or joining or lap welding or joining; or by laser welding. Combinations of these can also be used. Exemplary connections are illustrated in FIGS. 7A-7C. In FIG. 7A, system 300 includes a membrane 301 that is butt-welded at a first end 302 to a middle member 304 and lap welded at a second end 303 to an inner member 306. In FIG. 7B, system 310 includes a membrane 311 that is adhered at a first end 312 to a distal inner edge 315 of a middle member 314 and is retained at a second end 313 against an inner member 316 by an elastomeric ring 317 that applies force in a radially inward direction, squeezing the membrane 311 against the inner member 316. In FIG. 7C, system 320 includes a membrane 321 that is lap-welded at a first end 322 to a portion 325 of a middle member 324 that is proximal the distal end 329 of the middle member 324. A second end 323 of the membrane 321 is lap welded to an inner member 326 such that the second end 323 is distal to the first end 322. In some embodiments, the second end could be proximal the first end, while in other embodiments, the first and second ends could be at a point equally distal.

In some embodiments, for example, the embodiments of FIGS. 1-3, at least a retaining region 41 of the middle member 34 that constrains the stent 38 is constructed to have sufficient hoop strength to reduce and/or prevent the stent 38 from expanding until the middle member 34 is retracted. The retaining region 41 of the middle member 34 may be constructed from one or more polymers, such as, for example, PEBAX^{®}, Hytrel^{®}, Arnitel^{®}, nylon, polybutylene terephthalate (PBT), polyethylene terephthalate (PET), polyimides, and/or blends thereof.

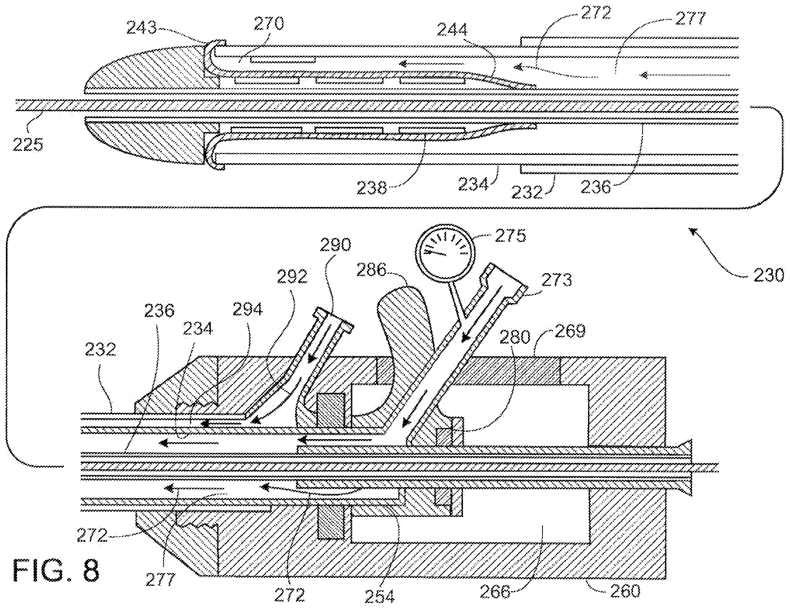

In certain embodiments, such as illustrated in FIG. 8, a delivery and deployment device 230 has a pull-back handle 280 that includes a fluid port 273, through which fluid can be introduced (e.g., via a fluid source, not here illustrated) into a lumen 277 between an inner member 236 and a middle member 234. The delivery and deployment device 230 has a membrane 244 that includes a region 243 that folds back upon itself to engage the distal end of the middle member 234. This folded arrangement results in the formation of a gap 270 between the membrane 244 and the middle member 234, which can function as a fluid chamber into which fluid (e.g., a liquid or a gas), represented by arrows 272, can be transported via the lumen 277. The fluid source can be for example, a syringe, compressor, gas tank, and/or an inflation device used, e.g., for angioplasty procedures. The fluid port 273 is located in the pull-back handle 280, to which a proximal end 254 of the middle member 234 is rigidly and sealingly attached. The fluid port 273 will travel with the pull-back handle 280 when it is moved longitudinally within a recess 266 of a handle 260 located at a proximal end of the device 230. The fluid flows into and optionally pressurizes the gap 270.

The fluid 272 may include a lubricating fluid, such as a lubricious hydrogel and/or saline, which can aid in reducing the potential frictional interactions between the middle member 234 and the membrane 244. In some embodiments, the fluid 272 may include a contrast agent (e.g., a radiopaque dye). In some embodiments, a volume of fluid 272 may be injected into the lumen 277 under a predetermined pressure which is maintained during the stent delivery process. The use of fluid 272 under pressure keeps the gap 270 between the middle member 234 and the membrane 244 open throughout the retraction process, effectively providing a liquid bearing effect and minimizing any sliding friction therebetween, as well as limiting the frictional forces resulting from the stent's tendency to push outward against the middle member 234. In addition, the pressure exerted by the fluid 272 against the membrane 244 can also maintain the membrane 244 over the stent 238 and provides the folded-over membrane 244 with a turgid-like state sufficient to retain a portion of the stent 238 thereunder in the reduced state until the membrane 244 is itself retracted.

Optionally, the system includes a pressure gauge 275 or other mechanism for monitoring and/or regulating the volume, flow rate, and/or pressure of the fluid 272 with in the system. A desired pressure of fluid 272 may be maintained within the chamber 270 by the use of any of a variety of devices such as stop-cocks and/or relief valves. The pressure is selected to provide the desired effect on the membrane 244 without risking rupturing the membrane 244 or inflating the gap 270 to a point at which the outer diameters significantly change. The pressure in some embodiments is regulated to be no less than about 0.5 atm. (e.g., no less than about 1, no less than about 1.5, or no less than about 2 atm.) and/or no more than about 2 atm. (e.g., no more than about 1.5, no more than about 1, or no more than about 0.5 atm.).

The device can further include a flushing port 290 for introducing a flushing fluid, indicated by arrows 292, into a lumen 294 between the middle member 234 and the outer member 232. The flushing fluid can also serve as a lubricant between the two members.

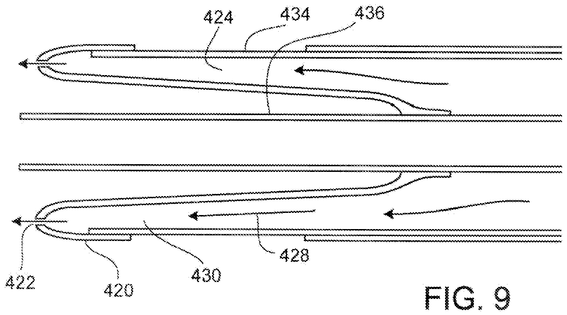

In certain embodiments, as illustrated in FIG. 9, a membrane 420 includes one or more weep holes 422 to permit fluid, represented by arrows 428, introduced into a lumen 424 between an inner member 436 and a middle member 434 to pass through the membrane 422. The number and size of the weep holes can be selected to permit a selected volume of fluid to pass through while maintaining a desired pressure in a gap 430 between the membrane 420 and the middle member 434. The membrane in certain embodiments can have at least one weep hole (e.g., at least two, three, four, five, ten, fifteen, or twenty weep holes) and/or no more than 25 weep holes (e.g., no more than twenty, fifteen, ten, five, four, three, or two weep holes). The weep holes can be circular or non-circular (e.g., oval, square, rectangular, slit-shaped, and/or random shaped). The weep hole or holes can in certain embodiments have a total cross-sectional area, summing all of the weep holes, of no less than 0.2 mm² (e.g., no less than 0.3 mm², no less than 0.4 mm², no less than 0.5 mm², or no less than 0.6 mm²) and/or no more than 0.75 mm² (e.g., no more than 0.7 mm², no more than 0.6 mm², no more than 0.5 mm², no more than 0.4 mm², or no more than 0.3 mm²). The individual weep hole or holes can in certain embodiments have a cross-sectional area of no less than 0.05 mm² (e.g., no less than 0.1 mm², no less than 0.2 mm², no less than 0.3 mm², or no less than 0.4 mm²) and/or no more than 0.5 mm² (e.g., no more than 0.4 mm², no more than 0.3 mm², no more than 0.2 mm², or no more than 0.1 mm²).

In certain embodiments, the outer surface of the endoprosthesis includes a coating, optionally including a therapeutic agent. The therapeutic agent can be a drug or other pharmaceutically active product, for example, a non-genetic agent, genetic agent, or cellular material. The term "therapeutic agent" includes one or more "therapeutic agents" or "drugs". Exemplary therapeutic agents or pharmaceutically active compounds are described in Phan et al., U.S. Patent No. 5,674,242; U.S.S.N. 11/165,949, filed on June 24, 2005, and entitled "Methods and Systems for Coating Particles"; and U.S. Published Application No. 2005/0192657 A1, published on September 1, 2005.

In some embodiments, the coating also includes a polymer. Exemplary polymers include biodegradable polymers (e.g., polylactic acid (PLA), polycaprolactone (PCL), and/or polyglyaxic acid (PGA)) and non-biodegradable polymers (e.g., styrene- isobutylene-styrene block copolymer (SIBS)). The polymer can protect a therapeutic agent contained in the coating such that the therapeutic agent is less susceptible to wearing off of the endoprosthesis before implantation.

In some embodiments the at least a portion of the stent may include a stent covering (e.g., the stent may be a stent graft). The covering may be constructed of a variety of materials, such as, for example, Dacron, PTFE, and/or expanded PTFE. In certain embodiments, the covering includes at least one therapeutic agent, which can be any of the therapeutic agents disclosed above.

While certain embodiments have been described, others are possible.

For example, the outer member can be a stiffening member (e.g., can be stiffer than the inner and/or middle members).

In some embodiments, the stiffness of one or more of the catheter members can be varied by changing the polymer durometers from the proximal end to the distal end.

In some embodiments, one or more of the catheter members can be formed of a multi-layer construction wherein one or more materials are layered, braided or otherwise combined to form the member.

In some embodiments, one or more of the catheter members may be provided with a liner (e.g., a PTFE liner) on either or both the interior and exterior faces thereof. Such a liner may be braided with an additional polymer.

In some embodiments, one or more of the catheter members are of the same or similar construction as a guide catheter.

In some embodiments, one or more of the catheter members and/or the membrane are at least partially constructed of a clear polymer. Such a clear polymer may be used, for example, to provide the member(s) with a substantially clear distal end region, which can allow for viewing the endoprosthesis while in a constrained state under the sheath.

In at least one embodiments, one or more of the catheter members and/or the membrane are coated for enhanced lubricity.

Other embodiments are in the claims.

## Claims

1. An implantable medical endoprosthesis delivery system (30; 230) comprising:
a first member (36; 236);
a second member (34; 234) slidably disposed around at least a portion of the first member (36; 236);
a third member (32; 232) disposed around at least a portion the second member (34; 234); and
a membrane (40; 244) connected to the first (36; 236) and second members (34; 234); **characterised in that** the third member (32; 232) is longitudinally fixed relative to the first member (36; 236).

2. The delivery system (30; 230) of claim 1, wherein the second member (34; 234) is slidably disposed over the first member (36; 236).

3. The delivery system (30; 230) of claim 1, wherein:
the second member (34; 234) has a delivery position configured so that, when an implantable medical endoprosthesis (38; 238) is positioned between the first (36; 236) and second members (34; 234), the membrane (40; 244) covers the endoprosthesis (38; 238);
a deployment position configured so that, when an implantable medical endoprosthesis (38; 238) is positioned between the first (36; 236) and second members (34; 234), the membrane (40) does not cover the endoprosthesis (38; 238); and
the distal end of the third member (32; 232) is proximal to the distal end of the second member (34; 234) when the second member (34; 234) is in the deployment position.

4. The implantable medical endoprosthesis delivery system (30; 230) of claim 1,
wherein a distal end of the third member (32; 232) is proximal to a distal end of the second member (34; 234).

5. The delivery system (30; 230) of claim 4, wherein the second member (34; 234) has a delivery position configured so that, when an implantable medical endoprosthesis (38; 238) is positioned between the first (36; 236) and second members (34; 234), the membrane (40; 244) covers the endoprosthesis (38; 238) and a first end and a second end of the membrane (40; 244) are proximal to the endoprosthesis (38; 238).

6. The delivery system (30; 230) of claim 4, wherein the second member (34; 234) has a delivery position configured so that, when an implantable medical endoprosthesis (38; 238) is positioned between the first (36; 236) and second members (34; 234), the membrane (40; 244) covers the endoprosthesis (38; 238) and a distal end of the second member (34; 234) is proximal to the endoprosthesis (38; 238).

7. The delivery system (30; 230) of claim 4, wherein the second member (34; 234) has a delivery position configured so that, when an implantable medical endoprosthesis (38; 238) is positioned between the first (36; 236) and second members (34; 234), the membrane (40; 244) covers the endoprosthesis (38; 238), a first end of the membrane (40; 244) is distal to the endoprosthesis (38; 238), and a second end of the membrane (40; 244) is proximal to the endoprosthesis (38; 238).

8. The delivery system (30; 230) of claim 4, wherein the membrane (40; 244) is capable of folding back over itself as the second member (34; 234) is moved proximally relative to the first member (36; 236).

9. The delivery system (30; 230) of claim 4, further comprising a fluid in a fluid lumen located between an outer surface of the first member (36; 236) and an inner surface of the second member (34; 234).

10. The delivery system (30; 230) of claim 4, wherein a proximal end of the second member (34; 234) is proximal a proximal end of the third member (32; 232).

11. The delivery system (30; 230) of claim 4, further comprising a handle (60) to which a proximal end of the first member (36; 236) and a proximal end of the third member (32; 232) are fixed.

12. The delivery system (30; 230) of claim 1, wherein the first (36; 236) and second members (34; 234) are configured so that an implantable medical endoprosthesis (38; 238) can be positioned therebetween.

## Patentansprüche

1. Implantierbares medizinisches Endoprothesen-Zuführungssystem (30; 230) mit:
einem ersten Element (36; 236);
einem zweiten Element (34; 234), das um mindestens einen Abschnitt des ersten Elements (36; 236) verschiebbar angeordnet ist;
einem dritten Element (32; 232), das um mindestens einen Abschnitt des zweiten Elements (34; 234) angeordnet ist; und
einer Membran (40; 244), die mit dem ersten (36; 236) und zweiten Element (34; 234) verbunden ist;
**dadurch gekennzeichnet, dass**
das dritte Element (32; 232) relativ zum ersten Element (36; 236) longitudinal fixiert ist.

2. Zuführungssystem (30; 230) nach Anspruch 1, wobei das zweite Element (34; 234) verschiebbar über dem ersten Element (36; 236) angeordnet ist.

3. Zuführungssystem (30; 230) nach Anspruch 1, wobei:
das zweite Element (34; 234) eine Zuführungsposition aufweist, die so konfiguriert ist, dass wenn eine implantierbare medizinische Endoprothese (38; 238) zwischen dem ersten (36; 236) und zweiten Element (34; 234) angeordnet ist, die Membran (40; 244) die Endoprothese (38; 238) bedeckt;
eine Einsatzposition, die so konfiguriert ist, dass wenn eine implantierbare medizinische Endoprothese (38; 238) zwischen dem ersten (36; 236) und dem zweiten Element (34; 234) angeordnet ist, die Membran (40) die Endoprothese (38; 238) nicht bedeckt; und das distale Ende des dritten Elements (32; 232) sich proximal zum distalen Ende des zweiten Elements (34; 234) befindet, wenn sich das zweite Element (34; 234) in der Einsatzposition befindet.

4. Implantierbares medizinisches Endoprothesen-Zuführungssystem (30; 230) nach Anspruch 1,
wobei sich ein distales Ende des dritten Elements (32; 232) proximal zu einem distalen Ende des zweiten Elements (34; 234) befindet.

5. Zuführungssystem (30; 230) nach Anspruch 4, wobei das zweite Element (34; 234) eine Zuführungsposition aufweist, die so konfiguriert ist, dass wenn eine implantierbare medizinische Endoprothese (38; 238) zwischen dem ersten (36; 236) und zweiten Element (34; 234) angeordnet ist, die Membran (40; 244) die Endoprothese (38; 238) bedeckt und sich ein erstes Ende und ein zweites Ende der Membran (40; 244) proximal zur Endoprothese (38; 238) befinden.

6. Zuführungssystem (30; 230) nach Anspruch 4, wobei das zweite Element (34; 234) eine Zuführungsposition aufweist, die so konfiguriert ist, dass wenn eine implantierbare medizinische Endoprothese (38; 238) zwischen dem ersten (36; 236) und zweiten Element (34; 234) angeordnet ist, die Membran (40; 244) die Endoprothese (38; 238) bedeckt und sich ein distales Ende des zweiten Elements (34; 234) proximal zur Endoprothese (38; 238) befindet.

7. Zuführungssystem (30; 230) nach Anspruch 4, wobei das zweite Element (34; 234) eine Zuführungsposition aufweist, die so konfiguriert ist, dass wenn eine implantierbare medizinische Endoprothese (38; 238) zwischen dem ersten (36; 236) und zweiten Element (34; 234) angeordnet ist, die Membran (40; 244) die Endoprothese (38; 238) bedeckt, sich ein erstes Ende der Membran (40; 244) distal zur Endoprothese (38; 238) befindet, und sich ein zweites Ende der Membran (40; 244) proximal zur Endoprothese (38; 238) befindet.

8. Zuführungssystem (30; 230) nach Anspruch 4, wobei die Membran (40; 244) imstande ist, sich auf sich selbst zurück zu klappen, wenn das zweite Element (34; 234) relativ zum ersten Element (36; 236) proximal bewegt wird.

9. Zuführungssystem (30; 230) nach Anspruch 4, das ferner ein Fluid in einem Fluidlumen aufweist, das sich zwischen einer Außenfläche des ersten Elements (36; 236) und einer Innenfläche des zweiten Elements (34; 234) befindet.

10. Zuführungssystem (30; 230) nach Anspruch 4, wobei sich ein proximales Ende des zweiten Elements (34; 234) proximal von einem proximalen Ende des dritten Elements (32; 232) befindet.

11. Zuführungssystem (30; 230) nach Anspruch 4, das ferner einen Griff (60) aufweist, an dem ein proximales Ende des ersten Elements (36; 236) und ein proximales Ende des dritten Elements (32; 232) befestigt sind.

12. Zuführungssystem (30; 230) nach Anspruch 1, wobei das erste (36; 236) und zweite Element (34; 234) so konfiguriert sind, dass dazwischen eine implantierbare medizinische Endoprothese (38; 238) angeordnet werden kann.

## Revendications

1. Système de mise en place d'une endoprothèse médicale implantable (30 ; 230) comprenant :
un premier élément (36 ; 236) ;
un deuxième élément (34 ; 234) disposé de manière coulissante autour d'au moins une partie du premier élément (36 ; 236) ;
un troisième élément (32 ; 232) disposé autour d'au moins une partie du deuxième élément (34 ; 234) ; et
une membrane (40 ; 244) raccordée au premier (36 ; 236) et au deuxième éléments (34 ; 234);
**caractérisé en ce que**
le troisième élément (32 ; 232) est fixé longitudinalement par rapport au premier élément (36 ; 236).

2. Système de mise en place (30 ; 230) selon la revendication 1, où le deuxième élément (34 ; 234) est disposé de manière coulissante sur le premier élément (36 ; 236).

3. Système de mise en place (30 ; 230) selon la revendication 1, où :
le deuxième élément (34 ; 234) présente une position de mise en place telle que la membrane (40 ; 244) couvre une endoprothèse médicale implantable (38 ; 238) quand l'endoprothèse (38 ; 238) est disposée entre le premier (36 ; 236) et le deuxième éléments (34 ; 234) ;
une position de déploiement telle que la membrane (40 ; 244) ne couvre pas une endoprothèse médicale implantable (38 ; 238) quand l'endoprothèse (38 ; 238) est disposée entre le premier (36 ; 236) et le deuxième éléments (34 ; 234) ; et où l'extrémité distale du troisième élément (32 ; 232) est proximale à l'extrémité distale du deuxième élément (34 ; 234) quand le deuxième élément (34 ; 234) est en position de déploiement.

4. Système de mise en place d'une endoprothèse médicale implantable (30 ; 230) selon la revendication 1, où une extrémité distale du troisième élément (32 ; 232) est proximale à une extrémité distale du deuxième élément (34 ; 234).

5. Système de mise en place (30 ; 230) selon la revendication 4, où le deuxième élément (34 ; 234) présente une position de mise en place telle que la membrane (40 ; 244) couvre une endoprothèse médicale implantable (38 ; 238) quand l'endoprothèse (38 ; 238) est disposée entre le premier (36 ; 236) et le deuxième éléments (34 ; 234), et qu'une première extrémité et une deuxième extrémité de la membrane (40 ; 244) sont proximales à l'endoprothèse (38 ; 238).

6. Système de mise en place (30 ; 230) selon la revendication 4, où le deuxième élément (34 ; 234) présente une position de mise en place telle que la membrane (40 ; 244) couvre une endoprothèse médicale implantable (38 ; 238) quand l'endoprothèse (38 ; 238) est disposée entre le premier (36 ; 236) et le deuxième éléments (34 ; 234), et qu' une extrémité distale du deuxième élément (34 ; 234) est proximale à l'endoprothèse (38 ; 238).

7. Système de mise en place (30 ; 230) selon la revendication 4, où le deuxième élément (34 ; 234) présente une position de mise en place telle que la membrane (40 ; 244) couvre une endoprothèse médicale implantable (38 ; 238) quand l'endoprothèse (38 ; 238) est disposée entre le premier (36 ; 236) et le deuxième éléments (34 ; 234), qu' une première extrémité de la membrane (40 ; 244) est distale à l'endoprothèse (38 ; 238), et qu' une deuxième extrémité de la membrane (40 ; 244) est proximale à l'endoprothèse (38 ; 238).

8. Système de mise en place (30 ; 230) selon la revendication 4, où la membrane (40 ; 244) est apte à se replier sur elle-même quand le deuxième élément (34 ; 234) est déplacé proximalement par rapport au premier élément (36 ; 236).

9. Système de mise en place (30 ; 230) selon la revendication 4, comprenant en outre un fluide dans une lumière de fluide située entre une surface extérieure du premier élément (36 ; 236) et une surface intérieure du deuxième élément (34 ; 234).

10. Système de mise en place (30 ; 230) selon la revendication 4, où une extrémité proximale du deuxième élément (34 ; 234) est proximale à une extrémité proximale du troisième élément (32 ; 232).

11. Système de mise en place (30 ; 230) selon la revendication 4, comprenant en outre une poignée (60) à laquelle sont fixées une extrémité proximale du premier élément (36 ; 236) et une extrémité proximale du troisième élément (32 ; 232).

12. Système de mise en place (30 ; 230) selon la revendication 1, où le premier (36 ; 236) et le deuxième éléments (34 ; 234) sont prévus de manière à permettre le positionnement d'une endoprothèse médicale implantable (38 ; 238) entre eux.
